# EUROPÄISCHE PATENTSCHRIFT

(11) **EP 3 186 218 B1**
(45) Veröffentlichungstag und Bekanntmachung des Hinweises auf die Patenterteilung: **24.10.2018**
(21) Anmeldenummer: 15733352.7
(22) Anmeldetag: 26.06.2015
(51) Int. Cl.: C07C 51/12, C07C 53/128, C07C 51/00, C07C 51/373

(54) **VERFAHREN ZUR HERSTELLUNG VON GEMISCHEN ENTHALTEND TERTIÄRE ISONONANSÄUREN AUSGEHEND VON 2-ETHYLHEXANOL**
METHOD FOR THE PRODUCTION OF MIXTURES CONTAINING TERTIARY ISONONANOIC ACIDS BASED ON 2-ETHYLHEXANOL
PROCÉDÉ DE PRODUCTION DE MÉLANGES CONTENANT DES ACIDES ISONANOÏQUES TERTIAIRES À PARTIR DE 2-ÉTHYLHEXANOL

(30) Priorität: 30.08.2014 DE 102014013123
(43) Veröffentlichungstag der Anmeldung: 05.07.2017
(73) Patentinhaber: OXEA GmbH, 46147 Oberhausen (DE)
(72) Erfinder: KOCKRICK, Kristina, 40595 Düsseldorf (DE); JOHNEN, Leif, 46286 Dorsten (DE); STRUTZ, Heinz, 47445 Moers (DE)
(86) Internationale Anmeldenummer: PCT/EP2015/001290
(87) Internationale Veröffentlichungsnummer: WO 2016/029978

(56) Entgegenhaltungen:
- WO-A1-2014/191090
- DE-A1- 2 406 223
- DE-B- 1 142 167
- HERBERT KOCH ET AL: "Über die Synthese verzweigter Carbonsäuren nach der Ameisensäure-Methode", JUSTUS LIEBIGS ANNALEN DER CHEMIE, Bd. 618, Nr. 1, 26. November 1958 (1958-11-26), Seiten 251-266, XP55204702, ISSN: 0075-4617, DOI: 10.1002/jlac.19586180127 in der Anmeldung erwähnt

## Beschreibung

Die vorliegende Erfindung betrifft die Herstellung von Gemischen enthaltend tertiäre Isononansäuren ausgehend von 2-Ethylhexanol durch Umsetzung von 2-Ethylhexanol mit einem Gemisch aus konzentrierter Ameisensäure und einer konzentrierten Brønstedsäure.

Unter tertiären Isononansäuren versteht man stellungsisomere aliphatische Carbonsäuren mit neun Kohlenstoffatomen im Molekül, in denen das der Carboxylgruppe benachbarte, α-ständige Kohlenstoffatom neben der Hauptkette noch zwei Alkylgruppen trägt. Dieses α-ständige Kohlenstoffatom trägt insgesamt vier Substituenten und wird häufig auch als quartäres Kohlenstoffatom bezeichnet. Ein technisch bedeutender Vertreter für eine solche stellungsisomere Isononansäure ist die 2,2,4,4-Tetramethylpentansäure. Ihre Herstellung erfolgt nach der Hydroxycarbonylierung oder der sogenannten Koch-Reaktion von technisch verfügbarem Di-isobuten mit Schwefelsäure bei erhöhtem Kohlenmonoxiddruck. (Ullmann's Encyclopedia of Industrial Chemistry, 6th. Ed., Vol. 6, p. 502-503, 2003 Wiley-VCH Verlag GmbH & Co. KGaA, Weinheim).

Technische Bedeutung besitzen ebenfalls sogenannte Versaticsäuren 9 der Momentive Specialty Chemicals Inc., deren Struktur wie folgt beschrieben werden kann: 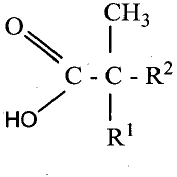 wobei R¹ und R² für Alkylgruppen stehen, die insgesamt 6 Kohlenstoffatome enthalten.

Neben der Verwendung der Dimeren des Isobutens werden auch die Dimeren des n-Butens als Ausgangsprodukt für die Herstellung von tertiären Isononansäuren beschrieben. Nach DE 199 06 518 A1 werden Octene aus der Dimerisierung von n-Butenen ebenfalls mittels der Koch-Reaktion unter Katalyse von starken Säuren mit Kohlenmonoxid und Wasser in die tertiären Isononansäuren überführt. Die Zusammensetzung des Isononansäuregemisches hängt von der Isomerenzusammensetzung des Di-n-Butens ab. Neben einem Isononansäuregemisch, das aus der Koch-Reaktion eines unfraktionierten Di-n-butens erhalten wird, beschreibt DE 199 06 518 A1 ebenfalls die Zusammensetzung von Isononansäuren auf Basis von Di-n-butenen, die als verzweigtere Kopffraktion und als weniger verzweigte Sumpffraktion bei der Fraktionierung von Di-n-buten anfallen. Die ausgewiesenen Isononansäuregemische enthalten 2-Ethyl-2-methylhexansäure in wechselnden Mengen als Hauptkomponente. Die veröffentlichte deutsche Patentanmeldung DE 10 2013 009 323 A1 betrifft ebenfalls die Herstellung eines Gemisches enthaltend tertiäre Isononansäuren ausgehend von einem Octengemisch als Ausgangsprodukt. Das Octengemisch wird durch Dehydratisierung von 2-Ethylhexanol gewonnen und anschließend in Gegenwart von sauren Katalysatoren mit Kohlenmonoxid bei erhöhtem Druck umgesetzt.

Die erhaltenen Gemische tertiärer Isononansäuren sind auf Grund ihrer hohen Verzweigung am α-ständigen Kohlenstoffatom wertvolle Zwischenprodukte in der industriellen organischen Chemie, die zu einer Vielzahl von Folgeprodukten für verschiedenste Anwendungsgebiete verarbeitet werden. Beispielsweise werden ihre Salze als Trocknungsbeschleuniger oder Sikkative für Lacke verwendet. Die Ester der tertiären Isononansäuren weisen eine hohe Hydrolyse- und Oxidationsbeständigkeit auf und ihre Polyolester, beispielsweise ihre Glykol- und Polyglykolester, werden als Hydraulikflüssigkeiten und Schmiermittel eingesetzt. Tertiäre Isononansäuren können ebenfalls in Vinylester derivatisiert werden, einmal durch Umsetzung mit Acetylen in Gegenwart von Zinksalzen bei Temperaturen von 200 bis 230°C oder durch die übergangsmetallkatalysierte Transvinylierungsreaktion mit einem Vinylester einer anderen Carbonsäure, häufig Vinylacetat oder Vinylpropionat (Ullmann's Encyclopedia of Industrial Chemistry, 6th. Ed., Vol. 38, p.70-73, 2003 Wiley-VCH Verlag GmbH & Co. KGaA, Weinheim). Vinylester besitzen als Monomerkomponente in Homo- und Copolymeren eine gewisse wirtschaftliche Bedeutung. Als Comonomere können sie die Eigenschaften von Polymeren, wie Polyvinylacetat, Polyvinylchlorid, Polystyrol oder Polyacrylsäureestern modifizieren. Die entsprechenden Copolymere lassen sich zu Anstrichen verarbeiten, die sich durch eine verbesserte Hydrolysebeständigkeit und eine geringere Feuchtigkeitsaufnahme auszeichnen. Auch für die Herstellung von Klebstoffen werden Vinylester von tertiären Isononansäuren eingesetzt. Tertiäre Isononansäuren können ebenfalls zu Glycidylestern umgesetzt werden, beispielsweise durch Reaktion mit Epichlorhydrin in Gegenwart von Natriumhydroxid nach dem in US 6,433,217 B1 beschriebenen Verfahren (Kirk-Othmer, Encyclopedia of Chemical Technology, 3rd Ed., Vol. 5, John Wiley & Sons 1979). Technisch werden Glycidylester von tertiären Carbonsäuren beispielsweise zur Modifizierung von Alkydharzen oder zur Herstellung von farbstabilen Beschichtungsmitteln für Außenanwendungen verwendet (Weissermel, Arpe, Industrielle Organische Chemie, VCH Verlagsgesellschaft, 3. Auflage, 1988, Seite 152; Ullmann's Encyclopedia of Industrial Chemistry, 6th Ed., Vol 24, Wiley-VCH-Verlag GmbH 2003, Seite 643).

Es ist ebenfalls bekannt, dass die Umsetzung von Olefinen zu einem Gemisch tertiärer Carbonsäuren im stark sauren Medium auch ohne Anwendung von Druck gelingt, wenn in Gegenwart von konzentrierter Ameisensäure als Kohlenmonoxidquelle gearbeitet wird. Diese auch als Koch-Haaf-Verfahren bekannte Variante kann ebenfalls mit Erfolg auch auf Alkohole als Ausgangsstoffe ausgedehnt werden. (Falbe, Carbon Monoxide in Organic Synthesis, Springer Verlag Berlin, Heidelberg, New York, 1970, Seiten 137-143; Angew. Chem., 70, 1958, Seite 311; Fette, Seifen, Anstrichmittel 59, 1957, Seiten 493-498). Nach Liebigs Ann. Chem. 618, 1958, Seiten 251-266 liefert die Umsetzung von 2-Methylbutanol neben der erwarteten 2,2-Dimethylbuttersäure noch weitere Säuren wie Trimethylessigsäure und C7-Säuren sowie einen erheblichen Anteil an höheren, hauptsächlich C11-Säuren, die von einem Penten-Dimer abgeleitet werden können.

Als Rohstoff für die industrielle Herstellung von tertiären Isononansäuren dient der C4-Schnitt aus der Dampfspaltung von Naphtha. Seine Verfügbarkeit im Vergleich zu den C2- und C3-Spaltprodukten kann durch die Bedingungen der Dampfspaltung gesteuert werden und richtet sich nach den Marktgegebenheiten.

Aus den C4-Spaltprodukten wird zunächst 1,3-Butadien durch Extraktion oder durch Selektivhydrierung in n-Butene entfernt. Das erhaltene C4-Raffinat, auch als Raffinat I bezeichnet, enthält überwiegend die ungesättigten Butene Isobuten, 1-Buten und 2-Buten sowie die hydrierten Produkte n-Butan und Isobutan. Aus dem Raffinat I wird im nächsten Schritt Isobuten entfernt, beispielsweise durch reversible protonenkatalysierte Wasseranlagerung zum tertiär-Butanol, und das erhaltene, isobutenfreie C4-Gemisch bezeichnet man als Raffinat II. Aus tertiär-Butanol kann durch Rückspaltung wieder Isobuten gewonnen werden (Weissermel, Arpe, Industrielle Organische Chemie, VCH Verlagsgesellschaft, 3. Auflage, 1988, Seiten 74-79). Ebenfalls kann das butadienfreie C4-Raffinat bei erhöhter Temperatur und unter Druck mit einem sauren suspendierten Ionenaustauscher in Kontakt gebracht werden. Isobuten oligomerisiert zu Di-isobuten, Tri-isobuten und in geringem Maße zu höheren Oligomeren. Die Oligomeren werden von den nicht reagierten C4-Verbindungen abgetrennt. Aus dem Oligomerisat kann dann Di-isobuten oder Tri-isobuten destillativ rein gewonnen werden. Durch die Dimerisierung von n-Butenen mit Isobuten wird in geringem Maße Codimer gebildet (Weissermel, Arpe, Industrielle Organische Chemie, VCH Verlagsgesellschaft, 3. Auflage, 1988, S. 77; Hydrocarbon Processing, April 1973, Seiten 171-173).

Di-isobuten, entweder hergestellt durch die Oligomerisierung von durch Rückspaltung erhaltenem reinem Isobuten oder gewonnen im Zuge der Aufarbeitung eines butadienfreien Raffinat I, wird anschließend in ein um ein C-Atom verlängertes C9-Derivat überführt. Dabei wird Di-isobuten nach der zuvor erwähnten Hydroxycarbonylierung oder Koch-Reaktion mit Kohlenmonoxid und Wasser in Gegenwart von Schwefelsäure in die hochverzweigte Isononansäure 2,2,4,4-Tetramethyl-1-pentansäure überführt. Aufgrund der zweifachen Alkylverzweigung an dem der Carboxylgruppe benachbarten Kohlenstoffatom wird diese stellungsisomere Isononansäure häufig auch als Neononansäure bezeichnet.

Auch die nach der Isobutenabtrennung im Raffinat II enthaltenen n-Butene werden technisch zu Butenoligomerisaten umgesetzt, aus denen isomere Octene abgetrennt werden, die über die Hydroxycarbonylierung in die entsprechenden Isononansäuren überführt werden (DE 199 08 320 A1; DE 199 06 518 A1). Die Oligomerisierung von n-Butenen wird technisch beispielsweise nach dem DIMERSOL-Prozess oder nach dem OCTOL-Prozess betrieben.

Vor dem Hintergrund, dass die Verfügbarkeit an Octenen basierend auf dem C4-Schnitt aus der Naphthaspaltung beschränkt ist und von den lokalen Standortbedingungen abhängt, ist es wünschenswert, weitere C8-Quellen auf Basis preiswert verfügbarer Großprodukte zu erschließen, die auf einfache Weise zu verschiedenen Standorten transportiert werden können. 2-Ethylhexanol steht als industrielles Großprodukt preiswert zur Verfügung, das ohne Probleme weitläufig vertrieben werden kann. 2-Ethylhexanol wird bekanntermaßen durch Hydroformylierung oder Oxo-Reaktion von Propylen zu n-Butyraldehyd mit nachfolgender alkalisch katalysierter Aldolkondensation zum 2-Ethylhexenal und anschließender Vollhydrierung zum 2-Ethylhexanol großtechnisch hergestellt (Ullmanns Encyklopädie der technischen Chemie, 4. Auflage, 1974, Verlag Chemie, Band 7, Seiten 214-215). Die Nutzung von 2-Ethylhexanol als C8-Quelle ermöglicht die Bereitstellung von tertiären Isononansäuren auf Basis von Propylen und mindert die Abhängigkeit von der C8-Verfügbarkeit auf Butenbasis.

Die Herstellung von tertiären Isonansäuren aus 2-Ethylhexanol wird in DE 1 142 167 beschrieben. Nach dem bekannten Verfahren wird eine Mischung aus 2-Ethylhexanol und Schwefelsäure unter Kohlenmonoxiddruck über Rieselkörper geleitet und überwiegend 2-Ethyl-2-methylhexansäure erhalten. Das bekannte Verfahren arbeitet bei hohen Drücken von 500 at.

Es wurde nun überraschenderweise gefunden, dass 2'-Ethylhexanol unter den Bedingungen der Koch-Haaf-Reaktion in Gegenwart eines Gemisches aus einer starken Brønstedsäure und Ameisensäure zu einem Gemisch aus isomeren Isononansäuren umgesetzt werden kann, das überwiegend 2-Ethyl-2-methylhexansäure, 2,2-Dimethylheptansäure und geringe Mengen an weiteren stellungsisomeren tertiären Isononansäuren enthält. Überraschenderweise werden Hochsieder, bei denen es sich um C17-Carbonsäuren aus der Dimerisierung von intermediär gebildeten Octenen mit nachfolgender Hydroxycarbonylierung handeln kann, nur in untergeordnetem Maße gebildet. Dies ist insofern unerwartet, da der Stand der Technik bei der Umsetzung von primären Alkoholen in größerem Maße über die Bildung von höheren Carbonsäuren, die sich aus der Dimerisierung der Kohlenstoffkette des Alkohols ableiten lassen, berichtet (Liebigs Ann. Chem. 618, 1958, Seiten 251-266).

Die vorliegende Erfindung besteht daher in einem Verfahren zur Herstellung eines Gemisches enthaltend tertiäre Isononansäuren ausgehend von 2-Ethylhexanol, dadurch gekennzeichnet, dass man
a) 2-Ethylhexanol bei einer Temperatur von 0°C bis 40°C mit einem Gemisch aus konzentrierter Ameisensäure und einer konzentrierten Brønstedsäure umsetzt, wobei pro mol 2-Ethylhexanol 2 bis 10 mol Ameisensäure eingesetzt werden und die konzentrierte Brønstedsäure in einer solchen Menge eingesetzt wird, die pro mol 2-Ethylhexanol 6 bis 90 mol an Protonen entspricht,
b) das gemäß Schritt a) erhaltene Reaktionsgemisch anschließend mit Wasser in Kontakt bringt, und
c) das gemäß Schritt b) gebildete Gemisch enthaltend tertiäre Isononansäuren abtrennt.

Vorzugsweise wird bei Schritt a) zunächst 2-Ethylhexanol und Ameisensäure gemischt und das Gemisch der vorgelegten konzentrierten Brønstedsäure zugeführt. Pro mol 2-Ethylhexanol werden 2 bis 10 mol, vorzugsweise 3 bis 6 mol Ameisensäure verwendet. Die konzentrierte Brønstedsäure wird in einer solchen Menge eingesetzt, die pro mol 2-Ethylhexanol 6 bis 90 mol, vorzugsweise 12 bis 40 mol an Protonen entspricht.

Unter dem Begriff Brønstedsäure versteht man die im Stand der Technik übliche Definition, nach der solche Verbindungen als Brønstedsäuren bezeichnet werden, die zur Abspaltung von Protonen fähig sind (Römpps Chemie-Lexikon, 8. Auflage, Franckh'sche Verlagshandlung, Stuttgart 1987, Band 5, Seiten 3651-3654). Als starke Brønstedsäure verwendet man beispielsweise Schwefelsäure, Fluorwasserstoffsäure, Phosphorsäure, Trifluormethansulfonsäure oder Fluorsulfonsäure und vorzugsweise Schwefelsäure. Die Brønstedsäure sowie die Ameisensäure werden als konzentrierte Säuren mit einer möglichst hohen Wirkkonzentration eingesetzt, wobei aber zweckmäßigerweise die kommerziell verfügbare konzentrierte Ameisensäure und Brønstedsäure benutzt wird. Im Allgemeinen stehen die konzentrierte Ameisensäure und die Bronstedsäuren mit einer Wirkkonzentration von 95 Gew.-% und höher kommerziell zur Verfügung (Ullmann's Encyclopedia of Industrial Chemistry, 5th. Edition, VCH Verlagsgesellschaft, Weinheim 1989, Vol. A12, Seite 26; Kirk-Othmer, Encyclopedia of Chemical Technology, 3rd. Edition, John Wiley & Sons, New York, 1983, Vol. 22, Seite 190).

In einer bevorzugten Ausgestaltung des erfindungsgemäßen Verfahrens werden kommerziell erhältliche konzentrierte Ameisensäure, üblicherweise mit einer Wirkkonzentration von 95 Gew.-% bis 100 Gew.-%, vorzugsweise von 95 Gew.-% bis 98 Gew.-% und kommerziell erhältliche konzentrierte Schwefelsäure, üblicherweise mit einer Wirkkonzentration von 96 Gew.-% bis 100 Gew.-%, vorzugsweise von 96 Gew.-% bis 98 Gew.-% verwendet, wobei pro mol 2-Ethylhexanol 3 bis 6 mol Ameisensäure eingesetzt werden. Dabei wird Schwefelsäure in einer solchen Menge eingesetzt, die 12 bis 40 mol an Protonen entspricht.

Die Umsetzung von 2-Ethylhexanol mit dem Gemisch aus Ameisensäure und Brønstedsäure im Schritt a) erfolgt bei Temperaturen zwischen 0°C und 40°C, vorzugsweise von 5°C bis 40°C und insbesondere von 5°C bis 20°C. Üblicherweise werden die Reaktionskomponenten über einen Zeitraum von 0,5 bis 2 Stunden zusammengeführt, in einer bevorzugten Ausgestaltung wird das Gemisch aus 2-Ethylhexanol und konzentrierter Ameisensäure über diesen Zeitraum der vorgelegten konzentrierten Brønstedsäure zugegeben. Nach Zugabe lässt man über einen Zeitraum von zweckmäßigerweise 20 bis 70 Stunden entweder bei Reaktionstemperatur isotherm, bei Eigentemperatur adiabatisch oder zunächst bei Reaktionstemperatur isotherm und anschließend bei Eigentemperatur adiabatisch nachreagieren, beispielsweise unter ständigem Rühren oder man vermeidet eine Bewegung des Reaktionsgemisches. Man lässt bei Atmosphärendruck oder bei erhöhtem Druck das Gemisch aus 2-Ethylhexanol, Ameisensäure und Brønstedsäure reagieren. Vorzugsweise arbeitet man unter Atmosphärendruck. Wird bei erhöhtem Druck gearbeitet, empfiehlt sich ein Überdruck von über Atmosphärendruck bis 1 MPa, vorzugsweise ein Überdruck von 0,1 bis 0,5 MPa. Der Überdruck kann durch Aufpressen von Kohlenmonoxid oder von einem Inertgas, beispielsweise Stickstoff, eingestellt werden. Den Schritt a) kann man auch als Carbonylierungsteilschritt auffassen.

Anschließend wird nach erfolgtem Carbonylierungsteilschritt das Reaktionsgemisch mit Wasser in Kontakt gebracht, beispielsweise in der Weise, dass man das Reaktionsgemisch auf ein äquivalentes Volumen Wasser oder Eis gibt. Den dabei ablaufenden Schritt b) kann man auch als Hydrolyseteilschritt auffassen. Das Inkontaktbringen mit Wasser kann bei dem Überdruck erfolgen, bei dem auch in dem Carbonylierungsteilschritt gearbeitet wurde, oder das Reaktionsgemisch aus dem Carbonylierungsteilschritt wird auf Atmosphärendruck entspannt und unter Atmosphärendruck mit Wasser in Kontakt gebracht. Wird bereits der Carbonylierungsteilschritt bei Atmosphärendruck durchgeführt, wird zweckmäßigerweise auch im Hydrolyseteilschritt bei Atmosphärendruck gearbeitet. Die dabei gebildete organische Phase, die das Gemisch enthaltend tertiäre Isononansäuren umfasst, wird von der wässrigen Phase getrennt und durch Wasserwäsche gereinigt.

Ohne zu sehr an mechanistische Überlegungen gebunden zu sein, kann angenommen werden, dass bei der Koch-Haaf-Variante durch die wasserentziehende Wirkung der konzentrierten Brønstedsäure, insbesondere der konzentrierten Schwefelsäure, Ameisensäure zu Kohlenmonoxid zersetzt wird. Weiterhin kann vermutet werden, dass 2-Ethylhexanol protoniert wird und unter Wasserabspaltung sich ein Carbeniumion bildet, das mit Kohlenmonoxid unter Bildung eines Acyliumkations weiterreagiert. Da die Reaktion unter Wasserentzug erfolgt, sind Ameisensäure und Brønstedsäure, insbesondere Schwefelsäure in konzentrierter Form einzusetzen, üblicherweise mit einem Wirkgehalt von 95 bis 98 Gew.-%. Ameisensäure und Brønstedsäuren in geringerer Konzentration sind aufgrund des zu hohen Wassergehaltes nicht zu empfehlen.

Das nach Schritt a) erhaltene Reaktionsgemisch wird anschließend gemäß Schritt b) mit Wasser in Kontakt gebracht, beispielsweise durch Aufgeben auf Eis. Durch diesen Hydrolyseteilschritt mit Wasser oder Eis wird das Acyliumkation abgesättigt. Bei dieser Reaktionsfolge aus Carbonylierungsteilschritt und anschließendem Hydrolyseteilschritt, die man zusammen auch als Hydroxycarbonylierung bezeichnen kann, bildet sich aus 2-Ethylhexanol ein Gemisch aus stellungsisomeren aliphatischen Isononansäuren, die an dem der Carboxylgruppe benachbarten Kohlenstoffatom zwei α, α-Alkylreste tragen und eine sogenannte Neo-Struktur besitzen. Man bezeichnet solche Isononansäuren auch als tertiäre Isononansäuren.

Der Carbonylierungsteilschritt und der nachfolgende Hydrolyseteilschritt finden in für chemische Prozesse üblichen Reaktoren statt, beispielsweise in einem Rührkessel oder in einer Rührkesselkaskade oder in einem Strömungsrohr, das als Leerrohr betrieben werden kann. Alternativ kann das Strömungsrohr mit statischen Mischern, wie Schüttungen, Packungen oder Einbauten versehen werden, die eine intensive Durchmischung der Reaktionslösung ermöglichen. Die beiden Schritte a) und b) werden separat betrieben, entweder kontinuierlich oder absatzweise. Es fällt ein Zweiphasengemisch aus einer wässrigen Phase und einer organischen Phase, die das gebildete Gemisch enthaltend tertiäre Isononansäuren umfasst, an. Gemäß Schritt (c) wird das gebildete Gemisch enthaltend tertiäre Isononansäuren abgetrennt, beispielsweise durch Phasentrennung. Anschließend wird das organische Produkt mit Wasser neutral gewaschen.

Bei dem erhaltenen Gemisch tertiärer Isononansäuren durch Umsetzung von 2-Ethylhexanol mit Ameisensäure in Gegenwart der konzentrierten Brønstedsäure liegen nach der gaschromatographischen Analyse gemäß DIN 51405 (FI.-%) als Hauptkomponente 2-Ethyl-2-methylhexansäure und als Nebenkomponente 2,2-Dimethylheptansäure vor sowie geringe Mengen an weiteren stellungsisomeren aliphatischen tertiären Isononansäuren. Der Anteil an 2-Ethyl-2-methylhexansäure und an 2,2-Dimethylheptansäure in Summe beträgt im Allgemeinen mehr als 90 mol-%, bezogen auf den Gesamtgehalt an stellungsisomeren aliphatischen tertiären Isononansäuren.

Überraschenderweise werden Nachlaufkomponenten, bei denen es sich vermutlich um höhere C17-Carbonsäuren handelt, die aus der Dimerisierung des C8-2-Ethylhexanolalkoholgerüstes mit nachfolgender Reaktion mit Kohlenmonoxid herrühren können, nur in einem untergeordneten Maße gebildet. Ihr Anteil in der Rohsäure liegt im Allgemeinen bei maximal 8% (FI.-%, gaschromatographisch gemäß DIN 51405).

Aus dem nach der Hydroxycarbonylierung abgetrennten Rohsäuregemisch wird mittels Destillation unter üblichen Bedingungen das gereinigte Gemisch stellungsisomerer aliphatischer tertiärer Isononansäuren gewonnen.

Das nach dem erfindungsgemäßen Verfahren hergestellte Gemisch enthaltend tertiäre Isononansäuren kann beispielsweise nach an sich bekannten Verfahren zur Herstellung von Derivaten wie den Vinylester, Glycidylester, Carbonsäureestern, Isononansäureanhydriden, Isononansäurehalogeniden oder Isononansäureamiden verwendet werden. Der Vinylester wird beispielsweise hergestellt durch Umsetzung der tertiären Isononansäuren mit Acetylen, vorzugsweise in Gegenwart von Zinksalzen bei Temperaturen von 200-230°C (G. Hübner, Fette, Seifen, Anstrichmittel 68, 290 (1966), Ullmanns Encyklopädie der technischen Chemie, 4. Auflage, 1983, Verlag Chemie, Band 23, Seiten 606-607) oder durch die sogenannte Transvinylierungsreaktion 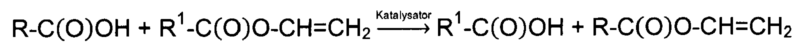 wobei R gleich C8 ist und R¹ häufig Methyl oder Ethyl bedeutet, so dass als Transvinylierungsreagenz beispielsweise Vinylacetat oder Vinylpropionat verwendet wird (Ullmanns Encyklopädie der technischen Chemie, 4. Auflage, 1983, Verlag Chemie, Band 23, Seiten 606-607). Um das chemische Gleichgewicht in Richtung des gewünschten Vinylesters zu drängen, verwendet man häufig einen Überschuss an dem Transvinylierungsreagenz R¹-C(O)-CH=CH₂ und entfernt gleichzeitig die gebildete Carbonsäure aus dem Reaktionsgemisch. Als Transvinylierungskatalysator eignen sich Verbindungen der Übergangsmetalle aus der Platingruppe Ruthenium, Osmium, Rhodium, Iridium, Palladium und Platin, insbesondere Palladium und Ruthenium, die modifiziert mit ein- oder mehrzähnigen Organostickstoff- oder Organophosphorliganden oder in unmodifizierter Form eingesetzt werden können. Ein solches Verfahren ist beispielsweise aus EP 0 497 340 A2 bekannt. In der in DE 10 2012 002 282 A1 beschriebenen kontinuierlichen Verfahrensvariante erfolgt die Umsetzung ohne Entzug eines Reaktionspartners und das abgeführte Reaktionsgemisch wird anschließend in seine Bestandteile aufgetrennt.

Das erhaltene Vinylisononanoat auf Basis tertiärer Isononansäuren eignet sich als Comonomer, beispielsweise in Polyvinylacetat, Polyvinylchlorid, Polystyrol oder Polyacrylsäureestern.

Aus dem erfindungsgemäß hergestellten Isononansäuregemisch kann ebenfalls der entsprechende Glycidylester, beispielsweise durch Umsetzung mit Epichlorhydrin in Gegenwart von Natriumhydroxid, nach an sich bekannten Verfahren hergestellt werden, der zur Modifizierung von Alkydharzen oder zur Herstellung von farbstabilen Beschichtungsmitteln dienen kann (Weissermel, Arpe, Industrielle Organische Chemie, VCH Verlagsgesellschaft, 3. Auflage, 1988, Seite 152; US 6433217; Ullmann's Encylopedia of Industrial Chemistry, Vol. 24, 6th Ed, Wiley-VCH-Verlag GmbH 2003, Seite 643).

Das erfindungsgemäß hergestellte Gemisch tertiärer Isononansäuren kann ebenfalls mit ein- oder mehrwertigen Alkoholen auf an sich bekannte Weise zu den entsprechenden Carbonsäureestern umgesetzt werden (Ullmanns Encyklopädie der technischen Chemie, 4. Auflage, 1976, Verlag Chemie, Band 11, Seiten 89-96), die in Schmierstoffzusammensetzungen, als Weichmacher für thermoplastische Polymere oder als Koaleszenzmittel in Dispersionsanstrichen verwendet werden können. Das erfindungsgemäß hergestellte Gemisch tertiärer Isononansäuren eignet sich ebenfalls für die Herstellung von öllöslichen Metallseifen, die als Sikkative oder Trockenstoffe verwendet werden (Ullmanns Encyklopädie der technischen Chemie, 4. Auflage 1983, Verlag Chemie, Band 23, Seiten 421-424).

Ebenso kann das erfindungsgemäß hergestellte Gemisch tertiärer Isononansäuren durch Umsetzung mit Halogenierungsmitteln wie Phosphorpentachlorid, Phosphoroxychlorid, Sulfurylchlorid oder Thionylchlorid in Isononansäurehalogenide derivatisiert werden, aus denen durch Umsetzung mit dem erfindungsgemäß hergestellten Gemisch tertiärer Isononansäuren Isononansäureanyhdrid oder durch Umsetzung mit anderen Carbonsäuren gemischte Anhydride zugänglich sind. Auch die Umsetzung von dem erfindungsgemäß hergestellten Gemisch tertiärer Isononansäuren mit Essigsäureanhydrid ergibt als Zwischenstufe das gemischte Anhydrid, das unter weiterer Säurezugabe und Essigsäureabspaltung in Isononansäureanhydrid oder in ein weiteres gemischtes Anhydrid umgewandelt werden kann (Ullmanns Encyklopädie der technischen Chemie, 4. Auflage, 1975, Verlag Chemie, Band 9, Seiten 145-146). Ausgehend von Isononansäurechlorid oder Isononansäureanhydrid können durch Umsetzung mit Ammoniak, primären oder sekundären Aminen die entsprechenden Isononansäureamide erhalten werden (Methoden der Organischen Chemie, Houben-Weyl, 4. Auflage, 1958, Georg Thieme Verlag, Stuttgart, Band XI/2, Seiten 10-14, 16-19). Entsprechend führt die Reaktion mit Alkoholen zu den Carbonsäureestern. (Methoden der Organischen Chemie, Houben-Weyl, 4. Auflage, 1952, Georg Thieme Verlag, Stuttgart, Band VIII, Seiten 547-549).

Aufgrund der zahlreichen Derivatierungsmöglichkeiten zu wertvollen Folgeprodukten werden tertiäre Isononansäuren im industriellen Maßstab in großen Mengen produziert. Das erfindungsgemäße Verfahren gestattet auf einfache Weise einen Zugang zu tertiären Isononansäuren ausgehend von 2-Ethylhexanol, das auch im technischen Maßstab betrieben werden kann.

In den folgenden Beispielen wird die Herstellung des Gemisches enthaltend stellungsisomere aliphatische tertiäre Isononansäuren durch Umsetzung von 2-Ethylhexanol mit konzentrierter Ameisensäure in Gegenwart von konzentrierter Schwefelsäure beschrieben.

### Beispiele

### Beispiel 1

In einem Glaskolben wurden14,6 mole Schwefelsäure in Form einer 96%-igen Schwefelsäure vorgelegt und mit Eis gekühlt. Anschließend wurde unter Eiskühlung ein Gemisch aus 4,9 mole Ameisensäure in Form einer 96%-igen Ameisensäure und 1 mol 2-Ethylhexanol so langsam zugetropft, dass die Temperatur der Reaktionsmischung 10°C nicht überstieg. Nach erfolgter Zugabe wurde für weitere 4,5 Stunden unter Eiskühlung gerührt und anschließend für weitere 16 Stunden bei Raumtemperatur gerührt. Im Anschluss wurde auf Eis gegossen. Nach Phasentrennung wurde die organische Phase mit Wasser neutral gewaschen und über Magnesiumsulfat getrocknet.

### Beispiel 2

Es wurde gemäß Beispiel 1 gearbeitet. Nach erfolgter Zugabe des Gemisches aus 2-Ethylhexanol und konzentrierter Ameisensäure zu der eisgekühlten konzentrierten Schwefelsäure ließ man noch 3 Stunden unter Eiskühlung rühren. Anschließend ließ man den Reaktionsansatz bei Raumtemperatur 64 Stunden lang stehen. Danach wurde auf Eis gegossen und die organische Phase abgetrennt. Die organische Phase wurde mit Wasser neutral gewaschen. Anschließend wurde Restwasser aus der organischen Phase durch Zentrifugieren abgetrennt.
Die gemäß Beispiel 1 und 2 erhaltenen Rohprodukte weisen folgende gaschromatographisch ermittelte Zusammensetzung (FI.-%, gemäß DIN 51405) auf.

Tabelle 1: Gaschromatographische Zusammensetzung des nach Beispiel 1 und 2 erhaltenen Gemisches stellungsisomerer aliphatischer tertiärer Isononansäuren

| ***Beispiel*** | ***1*** | ***2*** |
|---|---|---|
| Nachreaktion | 4,5 h Rühren unter Eiskühlung, 16 h Rühren bei Raumtemperatur | 3 h Rühren unter Eiskühlung, 64 h Stehenlassen bei Raumtemperatur |
| Aufarbeitung | Trocknen der organischen Phase nach Neutralwaschen durch Zugabe MgSO₄ | Abtrennung des Restwassers nach Neutralwaschen durch Zentrifugieren |
| Zusammensetzung laut GC (FI.-%, gemäß DIN 51405 | | |
| Vorlauf /Zwischenlauf | 0,8 | 0,3 |
| 2,2-Dimethylheptansäure | 26,4 | 17,4 |
| 2-Ethyl-2-methylhexansäure | 66,8 | 73,1 |
| Stellungsisomere aliphatische tertiäre Isononansäuren | 3,0 | 2,5 |
| Nachlauf | 3,0 | 6,7 |

Wie die Ergebnisse der Tabelle 1 zeigen, werden hochsiedende Nachlaufkomponenten nur in geringem Maße gebildet. Auch beschränkt sich die Isomerenverteilung auf wenige Konstitutionsisomere. 2-Ethyl-2-methylhexansäure wird als Hauptprodukt gebildet und 2,2-Dimethylheptansäure liegt als Nebenkomponente vor. Weitere stellungsisomere aliphatische tertiäre Isononansäuren werden nur in untergeordnetem Maße gebildet.

## Patentansprüche

1. Verfahren zur Herstellung eines Gemisches enthaltend tertiäre Isononansäuren ausgehend von 2-Ethylhexanol, **dadurch gekennzeichnet, dass** man
a) 2-Ethylhexanol bei einer Temperatur von 0°C bis 40°C mit einem Gemisch aus konzentrierter Ameisensäure und einer konzentrierten Brønstedsäure umsetzt, wobei pro mol 2-Ethylhexanol 2 bis 10 mol Ameisensäure eingesetzt werden und die konzentrierte Brønstedsäure in einer solchen Menge eingesetzt wird, die pro mol 2-Ethylhexanol 6 bis 90 mol an Protonen entspricht,
b) das gemäß Schritt a) erhaltene Reaktionsgemisch anschließend mit Wasser in Kontakt bringt, und
c) das gemäß Schritt b) gebildete Gemisch enthaltend tertiäre Isononansäuren abtrennt.

2. Verfahren gemäß Anspruch 1, **dadurch gekennzeichnet, dass** man als Brønstedsäure Schwefelsäure, Fluorwasserstoffsäure, Phosphorsäure, Trifluormethansulfonsäure oder Fluorsulfonsäure verwendet.

3. Verfahren gemäß Anspruch 1 oder 2, **dadurch gekennzeichnet, dass** man pro mol 2-Ethylhexanol 3 bis 6 mol Ameisensäure und die Brønstedsäure in einer solchen Menge einsetzt, die 12 bis 40 mol an Protonen entspricht.

4. Verfahren gemäß einem oder mehreren der Ansprüche 1 bis 3, **dadurch gekennzeichnet, dass** man konzentrierte Ameisensäure in einer Konzentration von 95 Gew.-% bis 98 Gew.-% verwendet.

5. Verfahren gemäß einem oder mehreren der Ansprüche 1 bis 4, **dadurch gekennzeichnet, dass** man konzentrierte Brønstedsäure in einer Konzentration von 96 Gew.-% bis 98 Gew.-% verwendet.

6. Verfahren gemäß einem oder mehreren der Ansprüche 1 bis 5, **dadurch gekennzeichnet, dass** man als konzentrierte Brønstedsäure konzentrierte Schwefelsäure in einer Konzentration von 96 Gew.-% bis 98 Gew.-% verwendet.

7. Verfahren gemäß einem oder mehreren der Ansprüche 1 bis 6, **dadurch gekennzeichnet, dass** man pro mol 2-Ethylhexanol 3 bis 6 mol konzentrierte Ameisensäure in einer Konzentration von 95 Gew.-% bis 98 Gew.-% und konzentrierte Schwefelsäure in einer Konzentration von 96 Gew.-% bis 98 Gew.-% verwendet, wobei die Schwefelsäure in einer solchen Menge eingesetzt wird, die 12 bis 40 mol an Protonen entspricht.

8. Verfahren gemäß einem oder mehreren der Ansprüche 1 bis 7, **dadurch gekennzeichnet, dass** man Schritt a) bei Temperaturen von 5°C bis 40°C, vorzugsweise 5°C bis 20°C durchführt.

9. Verfahren gemäß einem oder mehreren der Ansprüche 1 bis 8, **dadurch gekennzeichnet, dass** man Schritt a) bei Atmosphärendruck durchführt.

10. Verfahren gemäß einem oder mehreren der Ansprüche 1 bis 8, **dadurch gekennzeichnet, dass** man Schritt a) bei einem Überdruck von über Atmosphärendruck bis 1 MPa, vorzugsweise bei einem Überdruck von 0,1 bis 0,5 MPa durchführt.

## Claims

1. A process for producing a mixture comprising tertiary isononanoic acids proceeding from 2-ethylhexanol, **characterized in that**
a) 2-ethylhexanol is reacted at a temperature of 0°C to 40°C with a mixture of concentrated formic acid and a concentrated Brønsted acid, wherein 2 to 10 mol of formic acid are used per mole of 2-ethylhexanol and the concentrated Brønsted acid is used in an amount that corresponds to 6 to 90 mol of protons per mole of 2-ethylhexanol,
b) the reaction mixture obtained according to step a) is subsequently brought into contact with water, and
c) the mixture comprising tertiary isononanoic acids formed according to step b) is removed

2. The process as claimed in claim 1, **characterized in that** the Brønsted acid used is sulfuric acid, hydrofluoric acid, phosphoric acid, trifluoromethanesulfonic acid or fluorosulfonic acid.

3. The process as claimed in claim 1 or 2, **characterized in that** 3 to 6 mol of formic acid, and the Brønsted acid in an amount that corresponds to 12 to 40 mol of protons, are used per mole of 2-ethylhexanol.

4. The process as claimed in one or more of claims 1 to 3, **characterized in that** concentrated formic acid is used at a concentration of 95% by weight to 98% by weight.

5. The process as claimed in one or more of claims 1 to 4, **characterized in that** concentrated Brønsted acid is used at a concentration of 96% by weight to 98% by weight.

6. The process as claimed in one or more of claims 1 to 5, **characterized in that** the Brønsted acid used is concentrated sulfuric acid at a concentration of 96% by weight to 98% by weight.

7. The process as claimed in one or more of claims 1 to 6, **characterized in that** 3 to 6 mol of concentrated formic acid at a concentration of 95% by weight to 98% by weight and concentrated sulfuric acid at a concentration of 96% by weight to 98% by weight are used per mole of 2-ethylhexanol, wherein the sulfuric acid is used in an amount that corresponds to 12 to 40 mol of protons.

8. The process as claimed in one or more of claims 1 to 7, **characterized in that** step a) is carried out at temperatures of 5°C to 40°C, preferably 5°C to 20°C.

9. The process as claimed in one or more of claims 1 to 8, **characterized in that** step a) is carried out at atmospheric pressure.

10. The process as claimed in one or more of claims 1 to 8, **characterized in that** step a) is carried out at a positive pressure from above atmospheric pressure to 1 MPa, preferably at a positive pressure of 0.1 to 0.5 MPa.

## Revendications

1. Procédé de fabrication d'un mélange contenant des acides isononanoïques tertiaires à partir de 2-éthylhexanol, **caractérisé en ce que**
a) du 2-éthylhexanol est mis en réaction à une température de 0 °C à 40 °C avec un mélange d'acide formique concentré et d'un acide de Brønsted concentré, 2 à 10 moles d'acide formique étant utilisées par mole de 2-éthylhexanol et l'acide de Brønsted concentré étant utilisé en une quantité correspondant à 6 à 90 moles de protons par mole de 2-éthylhexanol,
b) le mélange réactionnel obtenu selon l'étape a) est ensuite mis en contact avec de l'eau, et
c) le mélange formé selon l'étape b) contenant des acides isononanoïques tertiaires est séparé.

2. Procédé selon la revendication 1, **caractérisé en ce que** l'acide sulfurique, l'acide fluorhydrique, l'acide phosphorique, l'acide trifluorométhanoïque ou l'acide fluorosulfonique est utilisé en tant qu'acide de Brønsted.

3. Procédé selon la revendication 1 ou 2, **caractérisé en ce que**, par mole de 2-éthylhexanol, 3 à 6 moles d'acide formique sont utilisées et l'acide de Brønsted est utilisé en une quantité correspondant à 12 à 40 moles de protons.

4. Procédé selon une ou plusieurs des revendications 1 à 3, **caractérisé en ce que** l'acide formique concentré est utilisé en une concentration de 95 % en poids à 98 % en poids.

5. Procédé selon une ou plusieurs des revendications 1 à 4, **caractérisé en ce que** l'acide de Brønsted concentré est utilisé en une concentration de 96 % en poids à 98 % en poids.

6. Procédé selon une ou plusieurs des revendications 1 à 5, **caractérisé en ce que** de l'acide sulfurique concentré en une concentration de 96 % en poids à 98 % en poids est utilisé en tant qu'acide de Brønsted concentré.

7. Procédé selon une ou plusieurs des revendications 1 à 6, **caractérisé en ce que**, par mole de 2-éthylhexanol, 3 à 6 moles d'acide formique concentré en une concentration de 95 % en poids à 98 % en poids sont utilisées et de l'acide sulfurique concentré en une concentration de 96 % en poids à 98 % en poids est utilisé, l'acide sulfurique étant utilisé en une quantité qui correspond à 12 à 40 moles de protons.

8. Procédé selon une ou plusieurs des revendications 1 à 7, **caractérisé en ce que** l'étape a) est réalisée à des températures de 5 °C à 40 °C, de préférence de 5 °C à 20 °C.

9. Procédé selon une ou plusieurs des revendications 1 à 8, **caractérisé en ce que** l'étape a) est réalisée à la pression atmosphérique.

10. Procédé selon une ou plusieurs des revendications 1 à 8, **caractérisé en ce que** l'étape a) est réalisée à une surpression de jusqu'à 1 MPa au-dessus de la pression atmosphérique, de préférence à une surpression de 0,1 à 0,5 MPa.
